Europäisches Patentamt

European Patent Office

Office européen des brevets

(11) Veröffentlichungsnummer: **0 501 032 A2**

# EUROPÄISCHE PATENTANMELDUNG

(21) Anmeldenummer: **91121527.5**

(51) Int. Cl.5: **C07H 15/04**

(22) Anmeldetag: **16.12.91**

(30) Priorität: **15.02.91 DE 4104640**

(43) Veröffentlichungstag der Anmeldung:
**02.09.92 Patentblatt 92/36**

(84) Benannte Vertragsstaaten:
**DE FR GB IT**

(71) Anmelder: **HÜLS AKTIENGESELLSCHAFT
Patentabteilung / PB 15 - Postfach 13 20
W-4370 Marl 1(DE)**

(72) Erfinder: **Ripke, Norbert, Dr.
Hellweg 28
W-4358 Haltern(DE)**

(54) Verfahren zur Herstellung hellfarbener Alkylpolyglycoside.

(57) Bei der Herstellung von Alkylpolyglycosiden mit einer Alkylgruppe mit 8 bis 24 C-Atomen durch Glycosidierung und Umglycosidierung von Sacchariden wird die Glycosidierung erfindungsgemäß in einem Verdampfer bei einer Säurezahl von 1 bis 10 mg KOH/g durchgeführt. Dabei werden farblich verbesserte Alkylpolyglycoside erhalten.

EP 0 501 032 A2

Die Erfindung betrifft ein Verfahren zur Herstellung von Alkylpolyglycosiden mit einer Alkylgruppe mit 8 bis 24 C-Atomen, bei dem Saccharide glycosidiert und die erhaltenen Produkte umglycosidiert werden.

Alkylpolyglycoside sind ungiftige und leicht abbaubare oberflächenaktive Stoffe. Sie werden deshalb als Wasch- und Reinigungsmittel und als Emulgatoren und Dispergatoren verwendet. Sie haben aber nur dann die gewünschten Grenzflächeneigenschaften, wenn die Alkylgruppen mindestens 8 C-Atome aufweisen.

Alkylpolyglycoside mit langkettigen Alkylresten können ganz oder teilweise aus nachwachsenden Rohstoffen hergestellt werden. Diese Alkylpolyglycoside gewinnen wegen ihrer interessanten Tensideigenschaften bei gleichzeitig sehr guter biologischer Abbaubarkeit zunehmend an Bedeutung. Für Anwendungen im Haushalt und im Kosmetikbereich müssen diese Produkte hohen ästhetischen Ansprüchen genügen. Man ist daher an Verfahren interessiert, nach denen Alkylpolyglycoside in transparenten, farblich schönen wäßrigen Lösungen hergestellt werden können.

Die Alkylpolyglycoside werden im allgemeinen aus Sacchariden und Alkoholen durch Glycosidierung und Umglycosidierung hergestellt. Bei einem zweistufigen Verfahren wird beispielsweise zunächst durch Glycosidierung mit n-Butanol ein n-Butylglycosid und daraus durch Umglycosidierung mit einem langkettigen Alkohol das gewünschte langkettige Alkylpolyglycosid hergestellt. Die Produkte, die man dabei erhält, sind jedoch, wenn keine zusätzlichen Maßnahmen ergriffen werden, dunkel gefärbt.

Die Farbe kann nach US 4 762 918 durch katalytische Hydrierung verbessert werden.

Bei der Herstellung von langkettigen Alkylsacchariden können nach US 4 465 828 auch die Hydroxylpolycarbonsäuren Citronensäure, Weinsäure und Äpfelsäure zur Farbverbesserung beitragen.

Nach EP 0 077 167 können bei der Umsetzung von Alkoholen mit Aldosen oder Ketosen Reduktionsmittel, wie hypophosphorige Säure oder schwefelige Säure, zugesetzt werden. Diese Additive sind praktisch an jeder Stelle im Prozeß von Vorteil. Durch sie wird die Farbe der Alkylglycoside verbessert.

Es sind auch vorbeugende Maßnahmen bekannt. So erhält man nach EP 0 102 558 farblich verbesserte $C_3$- bis $C_5$-Alkylglucoside, wenn man die Glucosidierung in Gegenwart eines Alkalisalzes einer Borsäure durchführt.

Nach EP 0 165 721 kann die Farbe der Produkte durch mehrstufige Bleichung mit Wasserstoffperoxid verbessert und durch Zusatz von Schwefeldioxid freisetzenden Verbindungen stabilisiert werden.

Die katalytische Hydrierung ist wegen der hohen Katalysatorkosten und der schwierigen Reaktionsführung problematisch. Die Wasserstoffperoxid-Bleichung erfordert bei großtechnischen Verfahren die Lagerung und Handhabung großer Mengen an Peroxid. Bei allen festen und flüssigen Farbverbesserern ist es außerdem schwierig, diese Mittel nach beendeter Reaktion quantitativ zu entfernen.

In EP 0 092 876 wird das Produkt nach beendeter Umglycosidierung und Neutralisation dadurch gereinigt, daß man die überschüssigen langkettigen Alkohole mit 12 bis 18 C-Atomen in einem Dünnschichtverdampfer abdestilliert. Bei diesem nachträglichen Reinigungsverfahren wird jedoch eine Farbstoffbildung während der Glycosidierung oder der Umglycosidierung nicht unterbunden.

Es bestand daher die Aufgabe, die genannten Nachteile des Standes der Technik zu vermeiden. Bei der Herstellung von hellfarbenen Alkylpolyglycosiden sollte eine Farbstoffbildung bereits während der Glycosidierung möglichst ohne fremde Hilfsstoffe, die später wieder abgetrennt werden müßten, unterdrückt werden.

Die Aufgabe wird erfindungsgemäß dadurch gelöst, daß man die Glycosidierung in einem Verdampfer bei einer Säurezahl (SZ) von 1 bis 10 mg KOH/g durchführt.

Es zeigte sich überraschend, daß eine schnelle Abtrennung des bei der Glycosidierung gebildeten und des zur Glycosidierung eingeführten Wassers erheblich zur Farbverbesserung der Alkylpolyglycoside beiträgt.

Geeignete Verdampfer für das erfindungsgemäße Verfahren sind beispielsweise Rohr-, Rohrbündel-, Fallfilm- und Dünnschichtverdampfer. Dabei werden Fallfilm- und Dünnschichtverdampfer vorzugsweise eingesetzt. Glycosidierungsmischungen können in diesen Verdampfern einer produktschonenden Flash-Verdampfung unterworfen werden.

Für die Glycosidierung werden im allgemeinen Saccharid, kurzkettiger Alkohol mit 1 bis 6 C-Atomen und saurer Katalysator gemischt und dann erwärmt, wobei die Reaktion unter Wasserbildung beginnt.

Als Saccharide können Monosaccharide, wie Glucose, Mannose, Gulose, Galaktose oder Talose, aber auch Di- und Oligosaccharide mit bis zu 10 Saccharideinheiten verwendet werden. Die Einheiten können 1,2-, 1,3-, 1,4- oder 1,6-verknüpft sein. Es können $\alpha$- oder $\beta$-Verknüpfungen vorliegen. Die Ketten können linear oder verzweigt sein. Vorzugsweise wird Glucose eingesetzt. Man kann auch wasserhaltige Produkte in die Reaktion einführen.

Als kurzkettige Alkohole können beispielsweise Ethanol, Propanol, Butanol, Pentanol, aber auch Glykole, wie Ethylenglykol und Propylenglykol verwendet werden.

Als saure Katalysatoren kann man Mineralsäuren,wie Schwefel- oder Phosphorsäure, einsetzen. Gut geeignet sind auch organische Säuren, wie beispielsweise Aryl-, Alkyl- oder Aralkylsulfonsäuren.

Während der Glycosidierung liegt die SZ vorzugsweise im Bereich von 1,5 bis 5 mg KOH/g.

Die Reaktion wird vorzugsweise bei einer Produkttemperatur von 60 bis 160 °C durchgeführt. Dabei wird ein Temperaturbereich von 80 bis 120 °C besonders bevorzugt. In einer besonderen Ausführungsform wird dafür gesorgt, daß die Produkttemperatur am Verdampferausgang 80 bis 120 °C beträgt. Die Reaktion kann bei Normaldruck, bei leichtem Unterdruck und auch bei Überdruck ausgeführt werden.

Die Verdampfer werden vorzugsweise bei einer Heiztemperatur von 120 bis 180 °C gefahren.

Die bei der Glycosidierung gewonnenen Alkylglycoside mit kurzkettigen Alkylgruppen werden anschließend durch Umglycosidierung mit langkettigen Alkoholen mit 8 bis 24 C-Atomen zu Alkylpolyglycosiden umgesetzt. Die Umglycosidierung kann nach bekannten Methoden erfolgen.

Die hier eingesetzten Alkohole können linear sein. Sie können aber auch Verzweigungen enthalten. Sie können gesättigt sein oder auch olefinische Doppelbindungen enthalten. Man kann natürliche oder synthetische Fettalkohole oder Fettalkoholgemische einsetzen. Als Beispiele seien Decanol, 10-Undecen-1-ol, Dodecanol, Myristylalkohol und Stearylalkohol genannt. Vorzugsweise enthalten die Alkohole 10 bis 18 C-Atome.

Die hergestellten Alkylpolyglycoside weisen einen mittleren Polymerisationsgrad von 1 bis 10 auf. Dabei werden niedrige mittlere Polymerisationsgrade von 1,3 bis 5 bevorzugt.

Das erfindungsgemäße Verfahren hat folgende Vorteile:
- Das Wasser wird schnell abgetrennt. Die Glycosidierung wird dadurch beschleunigt. Die Raum-Zeit-Ausbeute wird erhöht.
- Das Produkt wird thermisch weniger belastet. Produktschädigungen durch saure Hydrolyse werden erheblich reduziert. Man erhält deshalb farblich verbesserte Alkylpolyglycoside.

Obwohl das Reaktionsgemisch der Glycosidierung nicht neutralisiert ist, kommt es im Verdampfer nicht zu Anbackungen oder Verstopfungen.

Im allgemeinen wird bei der Durchführung des Verfahrens so vorgegangen, daß man die für die Glycosidierung frisch bereitete Mischung direkt auf einen als Verdampfer arbeitenden Wärmetauscher gibt. Die Reaktionsmischung wird dann 5- bis 20mal über den Wärmetauscher umgewälzt. Dadurch wird innerhalb kürzester Zeit Wasser effizient abgetrennt.

Die folgenden Beispiele sollen die Erfindung verdeutlichen.

Beispiel 1

In einem 5-l-Rührkessel mit aufgesetzter Kolonne und Wasserabscheider wird 1 kg Butylglucosid in 4 l Butanol bei 118 °C vorgelegt. Das Gemisch wird gerührt und gleichzeitig mit einem Volumenstrom von 22 l/h durch einen Fallfilmverdampfer (0,2 m² Fläche, 160 °C Öltemperatur) gepumpt und in den Rührkessel zurückgeleitet. Zu dem umgewälzten Produkt werden vor dem Eintritt in den Verdampfer pro Stunde 1 l Glucosesirup (60%ig in Wasser), 12,5 g $H_2SO_4$ und 3 l Butanol über einen Statikmischer zugegeben.

Produkttemperatur am Ausgang des Verdampfers: 116 °C
SZ im Rührkessel: 2 mg KOH/g

Butanol und Wasser werden abdestilliert und am Wasserabscheider getrennt. Butanol wird zurückgeführt.

Im Ablauf des Verdampfers liegt der Wassergehalt im Produkt bereits unter 1 %.

Nach einer Reaktionszeit von 1,25 Stunden bei 118 °C (Produkttemperatur im Kessel) und Normaldruck ist die Reaktion beendet.

Iodfarbzahl (IFZ) von Butylglucosid: 60 bis 80 (aus einer 35%igen Lösung in Butanol)

Zur anschließenden Umglycosidierung werden 2 kg Butylglucosid (IFZ: 60 bis 80) und weitere 12,5 g $H_2SO_4$ bei 116 °C und 400 hPa mit 3 l einer Mischung aus 70 % Dodecanol und 30 % Tetradecanol umgesetzt. Butanol wird destillativ entfernt. Nach ca. 2 Stunden ist die Reaktion beendet.

Danach wird neutralisiert, worauf überschüssiger Alkohol destillativ entfernt wird. Der erhaltene Feststoff wird in Wasser gelöst und mit Ozon gebleicht. Man erhält ein hellfarbenes Alkylpolyglucosid.

Mittlerer Polymerisationsgrad: 1,65
IFZ: 3 bis 5 (aus einer 50%igen wäßrigen Lösung)

Vergleichsbeispiel A

Es wird wie in Beispiel 1 verfahren. Die Glucosidierung erfolgt jedoch in einem 10-l-Rührkessel mit Wasserabscheider in 4 Stunden bei 118 °C, wobei aus dem gerührten Ansatz heraus destilliert wird.

IFZ von Butylglucosid: 100 bis 200
(aus einer 35%igen Lösung in Butanol)

Das mit diesem Butylglucosid gemäß Beispiel 1 hergestellte Alkylpolyglucosid hat folgende Eigenschaften:
Mittlerer Polymerisationsgrad: 1,65

IFZ: 20 (aus einer 50%igen wäßrigen Lösung)

**Patentansprüche**

1. Verfahren zur Herstellung von Alkylpolyglycosiden mit einer Alkylgruppe mit 8 bis 24 C-Atomen durch Glycosidierung und Umglycosidierung von Sacchariden,
dadurch gekennzeichnet,
daß man die Glycosidierung in einem Verdampfer bei einer Säurezahl von 1 bis 10 mg KOH/g durchführt.

2. Verfahren nach Anspruch 1,
dadurch gekennzeichnet,
daß man die Glycosidierung in einem Fallfilm- oder Dünnschichtverdampfer durchführt.

3. Verfahren nach Anspruch 1,
dadurch gekennzeichnet,
daß eine Säurezahl von 1,5 bis 5 mg KOH/g eingestellt wird.

4. Verfahren nach Anspruch 1,
dadurch gekennzeichnet,
daß die Glycosidierung in einem auf 120 bis 180 °C aufgeheizten Verdampfer durchgeführt wird.

5. Verfahren nach Anspruch 1,
dadurch gekennzeichnet,
daß die Produkttemperatur während der Glycosidierung 60 bis 160 °C beträgt.

6. Verfahren nach Anspruch 5,
dadurch gekennzeichnet,
daß die Produkttemperatur 80 bis 120 °C beträgt.